# NEW EUROPEAN PATENT SPECIFICATION

(11) **EP 0 574 461 B2**
(45) Date of publication and mention of the opposition decision: **22.11.2006**
(45) Mention of the grant of the patent: 17.05.2000
(21) Application number: 92905810.5
(22) Date of filing: 04.03.1992
(51) Int. Cl.: A61K 39/395, C07K 16/00

(54) **HIGH AFFINITY HUMANIZED MONOCLONAL ANTIBODIES**
HOCHAFFINE HUMANISIERTE MONOKLONALE ANTIKOERPER
ANTICORPS MONOCLONAUX HUMANISES A HAUTE AFFINITE

(30) Priority: 04.03.1991 GB 9104498
(43) Date of publication of application: 22.12.1993
(73) Proprietor: KS BIOMEDIX LTD., Esher, Surrey KT10 9QY (GB)
(72) Inventor: TAN, Kim, Sze 52 Farnham Road, Surrey GU2 5PE (GB)
(74) Representative: Chapman, Paul William
(86) International application number: PCT/GB1992/000384
(87) International publication number: WO 1992/015699

(56) References cited:
- NATURE, Vol. 349, 1991 Greg Winter et al: "Man-made antibodies ", see page 293 - page 299
- PROC.NATL.ACAD.SCI., Vol. 86, 1989 Cary Queen et al: "A humanized antibody that binds to the interleukin 2 receptor ", p 10029-10033
- JOURNAL OF ENDOCRINOLOGY, Vol. 126, 1990 D.J. Groves et al: "The preparation of an ovine monoclonal antibody to progesterone ", see page 217 - page 222
- Dialog Information Services, File 155, Medline, Dialog accession no. 07588889, Medline accession no 91107889, Kuzmanoff KM et al: "Isolation of monoclonal antibodies monospecific for bovine alpha-lactalbumin", J Dairy Sci Nov 1990, 73 (11) p 3077-83
- Dialog Information Services, File 155, Medline, Dialog accession no. 07345350, Medline accession no.90252350, Williams DJ et al: "Quantitation of bovineimmunoglobulin isotypes and allotypes using monoclonal antibodies", Vet Immunol Immunopathol Mar 1990, 24 (3) p 267-83
- Dialog Information Services, File 155, Medline, Dialog accession no. 07625649, Medline accession no.91144649, Smith TW: "Review of clinical experience with digoxin immune Fab (ovine).", Am J Emerg Med Mar 1991, 9 (2 Suppl 1) p 1-6
- Vet. Immunol. Immunopathol.,23:1-14 (1989)
- Science,229: 1202-1207 (1985)
- Am. J. Emergency Med., 9, 2 suppl.1 (1991)
- Abstracts Program of The Second Annual IBC International Conference on Antibody Engineering (December 1991)

## Description

### Field of the invention

This invention relates to monoclonal antibodies having high affinity, and to their therapeutic use.

### Background of the invention

Murine and rat monoclonal antibodies are widely available, but are generally unsuitable for therapeutic use in humans owing to the human antibody response to foreign proteins. In order to overcome this problem, it has been proposed to "humanise" rodent monoclonals, by combining the variable region of the monoclonal with the constant region derived from human immunoglobulin. In order to produce them in bulk the gene for a chimeric or other antibody of this type can be transfected into and expressed by myeloma cells or any other host cell line.

Another disadvantage associated with rodent antibodies is their low specificity and affinity. The affinity constant (Ka) of a, say, murine monoclonal may be up to 10¹⁰. Humanisation will often reduce the Ka value, but the original value may be substantially retained, by careful choice of the materials and experimental conditions. This has been the approach to high affinity monoclonals intended for therapeutic use in humans.

Flynn et al, Immunology 69 (1990) 1-7, describe production of an ovine monoclonal antibody from a sheep/mouse heterohybridoma fusion partner against synthetic peptides of the foot-and-mouth virus. No data are presented either in terms of the stability of the line or levels of antibody secreted, although it is noted that the lines produced had no or poor neutralisation/protection properties. Possible reasons for this are identified as (i) unique epitopes are not involved in neutralisation/ protection, (ii) antibody levels in the supernatants are too low, and (iii) not enough monoclonals were tested against these determinants.

Groves et al, J. Endocrinol. 126 (1990) 217-222, describe a stable high-affinity ovine monoclonal against progesterone following fusion with the murine NS1 myeloma, although a parallel fusion was run with their heterohybridoma fusion partner which had resulted in higher fusion efficiencies than the NS1 cells. This particular line has been stable for 28 months and secretes 5-10 µg/10⁶ cells/24 hours. This compares favourably with the earlier work reported by Groves et al, Res. Vet. Sci. 43 (1987) 253-256, where a similar NS1 x sheep fusion resulted in two cell lines: one stopped secreting after two months, but the other produced 2.5 ng/ml at confluence, 4 months post-fusion, after four subcloning steps.

WO 91/09966 is in the state of the art according to Article 54(3) EPC. It discloses a CDR-grafted antibody heavy chain having a variable region domain comprising acceptor framework and donor antigen binding regions at specific positions. The CDR-grafted antibodies are preferably humanised antibodies, having non-human acceptor frameworks. The antibodies are generally derived from rodents.

### Summary of the Invention

A chimeric monoclonal antibody according to the present invention comprises a monoclonal antibody according to claim 1. Further, for the purposes of producing such an antibody, novel heterologous DNA comprises a chimeric gene including variable region DNA encoding the non rodent monoclonal antibody heavy and light chains and constant region human DNA. Also provided by the invention is an antibody fragment according to claim 5.

An antibody of the invention may be produced by techniques analogous to those known for the production and chimerisation of murine monoclonals. The product, however, can have much higher affinity.

### Description of the Invention

Various techniques can be applied, in order to produce antibodies in accordance with the present invention. The product is predominantly of human origin, such that it can be used satisfactorily in a method of therapy practised on a human.

A whole antibody comprises heavy and light chains, and constant and variable regions. The variable regions comprise a variable framework and hypervariable regions within which the antigen-binding sites are located. The invention includes antibody fragments within its scope, which are F(ab')₂ or Fab fragments, provided that at least part thereof is derived from human immunoglobulin.

More specifically, a "chimeric" whole antibody of the invention comprises the high-affinity antibody variable region and the constant region of human immunoglobulin. In all cases, the product is a combination of the high-affinity antibody and human antibody sequences.

A product of the invention may be prepared by a process involving essentially three steps. Firstly, a suitable animal is immunised using an antigen, and B cells secreting an antibody to that antigen are obtained. Secondly, high-affinity monoclonal antibodies and the genes coding for them are obtained. Thirdly, these antibodies are humanised.

The animal that is subjected to immunisation is not a rodent, but is a mammal chosen to give higher affinity antibodies. Suitable animals are rabbits, cows and, for the purposes of further illustration, sheep. Immunisation of sheep gives good immunogenic response to a variety of antigens. Their size and life-span means that they can be given antigen via a number of routes of administration, and over a longer period, than rodents.

As illustrated below, in Example 1, the high-affinity monoclonal antibody may be obtained by classical hybridoma technology, comprising the fusion of the B cells secreting high-affinity ovine or cow antibodies with myeloma cells, and selection of resultant hybridomas. Alternatively, the B cells from the relevant species can be plated out, selected and amplified, e.g. by using the polymerase chain reaction; phage recovery may also be used, to obtain mRNA from selected lymphocytes, and thus the antibody genes.

Chimerisation follows. The monoclonal antibody that is used in the present invention for this purpose of chimerisation preferably has an affinity constant of at least 10¹², more preferably at least 5 x 10¹², and most preferably at least 10¹³, e.g up to 10¹⁴, 10¹⁵ l/mol or higher.

Various chimerisation techniques may be used, all of which involve the assembly of sequences from the high-affinity antibody and sequences from human antibodies. For example a chimeric whole antibody of the invention comprises the variable region of the former and the constant region of the latter. Techniques for producing chimeric whole antibodies are known.

Recombinant technology, for the purposes of producing an antibody according to the invention, may comprise a first step in which a large number of whole ovine or other cow antibody molecules are sequenced from the hinge region upwards, e.g. for a range of sheep IgG1's. This may be done either by isolation of mRNA from a number of B cell clones, perhaps using PCR and thus sequencing the DNA, or by sequencing IgG1 affinity-purified polyclonal antibodies or a large number of monoclonals, if available. The sequences can then be used to define the boundary between the constant, variable and hypervariable regions for both heavy and light chains. Structural modelling can then be used, for the purposes of comparison with human and mouse constant regions, to determine how much of the high-affinity monoclonal sequence, i.e. the variable region, to be added to the human constant gene, to give the whole product.

An antibody of the invention may be used in therapy. It is likely to be of particular value in the treatment of cancer, inflammation, e.g. rheumatoid arthritis, and septic shock, following organ transplant, in immunomodulation, for passive immunotherapy in the treament of viruses, and to provide antibodies against bacteria. For this purpose, the antibody may be formulated into a suitable composition with a physiologically-acceptable excipient, diluent or carrier.

Particular advantages of high affinity antibodies for therapy are:
(i) Affinity is related to biological response. The higher the affinity, the better the response is likely to be.
(ii) Higher affinity antibodies will result in more rapid binding of the antibodies to the target cells or more rapid immunoneutralisation. This means that there will be more of the antibodies concentrated at the target sites, directly leading to better localisation of antibody or antibody conjugate, by reducing the non-specific binding to other sites. This is an important consideration in cancer treatment, where binding to the tumour is desired at the expense of binding to other tissues such as kidney, bone marrow and liver.
(iii) Higher affinity means that less antibodies can be used per dosage, leading to more economically viable therapies. This is a relevant consideration where competition reactions are involved, e.g. binding with a virus or lymphokine rather than the virus or lymphokine binding with a cell surface or receptor.

The following Example 1 illustrates the preparation of an ovine monoclonal antibody having high affinity ("Guildhay" refers to Guildhay Antisera, Guildford, United Kingdom).

### Example 1

### PREPARATION OF HETEROMYELOMA FUSION PARTNER SFP1

SFP1 is a sheep heteromyeloma fusion partner derived originally from the NSI cell line. The line secretes neither murine nor ovine immunoglobulins. The SFPI cells have been treated with 8-azoguanine or 6-thioguanine, cloned by limiting dilution, and checked for HAT sensitivity. The cell line has not reverted to HAT resistance over a period of 3 years.

### 1. RIA for Ovine anti-T3 antibodies

The level of antibody secretion was unknown, possibly sub-nanogram quantities initially. The RIA for screening culture supernatant needed to be sensitive and was adapted from a routinely used T3 assay. Briefly, culture supernatants were incubated with iodinated T3 and bound tracer separated using a PEG-second antibody procedure.

All dilutions were made in 0.05 M barbitone buffer (pH 8.6) containing 0.1% RIA Grade BSA, (Sigma). 100 µl iodinated T3 7.4 x 10⁵ S⁻¹/ml (20 µCi/ml)[>4.44 x 10⁷ s⁻¹/µg (>1.2 mCi/µg) T3, Amersham International] was added to 100 µl 8-anilino-1-naphthalenesulphonic acid (4 mg/ml), 200 µl culture supernatant, 100 µl donkey anti-sheep immunoglobulin (Guildhay) and 75 µl tissue culture medium. An aliquot of sheep polyclonal antibody to T3 (Guildhay) giving 30% binding of total counts was included in the screening assay as a positive control. Tubes were vortexed and incubated for 2 hours at 37°C or overnight at 4°C before separation by the addition of 500 µl 6% w/v PEG 6000 (BDH), vortexing, and centrifuging for 30 min at 3000 rpm. The pellets were counted for 60 sec.

### 2. Ovine IgG ELISA

200 µl of a 7.5 µg/ml solution of affinity-purified donkey anti-sheep immunoglobulin (Guildhay) pretreated to remove antibodies cross-reacting with immunoglobulins present in foetal calf serum (FCS), in bicarbonate buffer (pH 9.6), were adsorbed onto 96-well microtitre plates (Nunc Maxisorp) for 2 hours at 37°C or overnight at 4°C. The plates were washed with PBS containing 0.1% gelatin and 0.05% Tween^{®} 20, pH 7.4 (PBSGT). 200 µl tissue culture supernatant or sheep gamma globulin standards (Sigma), 1-1000 ng/ml diluted in tissue culture medium, were added to the wells and incubated for 1 hour at 37°C. The plates were washed with PBSGT and 200 µl horseradish peroxidaselabelled donkey anti-sheep immunoglobulin (Guildhay) was added to the wells. Plates were incubated for 30 min at 37°C and washed with PBSGT.

200 µl substrate, 3,3',5,5'-tetramethylbenzidine (TMB) (Boehringer-Mannheim) in pH 5.5 citrate phosphate buffer + 0.04% H₂O₂ was added, and the colour allowed to develop for 30 min at 37°C. The reaction was stopped by the addition of 50 µl 2.5M H2S04, and the absorbence was read at 450 nm on Titertek Multiskan Plus^{®} (Flow Labs).

### FUSION

The lymphocytes for fusion were obtained from the spleen of a sheep which had been repeatedly immunised with T3-BSA conjugates over a period of 15 years, in order to obtain polyclonal antiserum to T3. The final boost was 1 week prior to fusion. A small area of spleen was teased apart to produce a cell suspension (large clumps of undissociated cells were discarded). The cell suspension was washed and spun twice in RPMI (no FCS or supplements). No further procedures, i.e. mitogens, enrichments or pre-culture, were performed prior to fusion.

Parallel fusions were performed using heteromyeloma fusion partner SFPI and murine NS2 myeloma. Fusions were performed in a ratio of 8 spleen to 1 myeloma cell, with a total cell number of 9 x 10⁸ in each case, using 1 ml 50% PEG 1500 (BDH) in RPMI over 2 minutes followed by slow dilution with 20 ml RPMI over a further 8 minutes. The fusions were plated out as follows:-
i. 20% FCS, RPMI, pyruvate (1 mm), glutamine (2 mm), HAT (Gibco) plus mouse spleen feeders.
ii. 20% FCS, RPMI, pyruvate, glutamine, HAT + no feeders.
iii. 10% FCS, 10% sheep serum, pyruvate, glutamine, HAT plus feeders.
iv. 10% FCS, 10% sheep serum, pyruvate, glutamine, HAT, no feeders.

Each fusion was in 3 x 96-well plates of each of the 4 parameters (24 x 96 wells total). During the first week, penicillin and streptomycin were added to the cultures as the spleen was removed and transported in non-sterile conditions, (transit time approximately 2 hours). 14 days post-fusion HAT was replaced by HT.

### RESULTS

The number of clones derived from the various treatments can be seen in Table 1. It is clear that more hybridoma cell lines resulted from the SFP1 cross (total 634) than NS2 (total 634). Though not so significant, mouse feeder cells appear to be beneficial in this system in terms of the number of hybridomas produced. The sheep serum did not increase the number of hybridomas produced, despite indications to the contrary in the literature.

Two months post-fusion, 57 lines were still weakly positive; one line was chosen for further study since it was considerably stronger than the others. This originated from an SFP1 x spleen cross grown in 20% FCS with no feeder cells. The resultant cell line 17C6 was visible under the microscope 11 days post-fusion (earliest lines visible 4 days post-fusion) but was not considered large enough to screen until 22 days post-fusion. Although strongly positive for T3 antibodies at this stage, by RIA it was not confluent enough to transfer to 2 ml wells for a further 2 weeks, when HAT in the medium was replaced by HT. The line was subcloned 33 days post-fusion (levels of antibody secretion had not diminished) and again re-subcloned 3 months post-fusion. Despite this initial very slow growth rate, the rate increased such that the line currently divides at rates comparable with conventional murine and human lines, i.e. approximately 26 hours.

Table 2 shows the effect of FCS and lamb serum concentrations on antibody production. Other experiments investigating the effects of lamb serum and mixtures of lamb and FCS or FCS alone over 5-20% range on exponentially-growing cultures showed that, after 1 week in culture, levels of T3 binding activity were equal for FCS over the range tested with approximately 85% of available T3 bound (confluent cultures would bind 100%). Over the same range, in lamb serum, approximately 45% of T3 was bound whereas the mixture of FCS and lamb serum showed an intermediate 55% binding after 1 week. After 48 hours growth, it appeared that lamb serum was best for the cell line.

### ANTIBODY CHARACTERISTICS

The association constant Kₐ for the sheep monoclonal antibody 17C6 was found to be 2.6 x 10¹³ L/mol which compares well with 1.39 x 10¹³ L/mol for serum from an early bleed and 2.13 x 10¹³ L/mol from the best polyclonal bleed (4 months prior to fusion). The cross-reactivities of these three antibodies are shown in Table 3. standard curves for the best clonal antiserum and monoclonal 17C6 were similar.

17C6 was sub-classed using monoclonal ovine IgGI, IgG2, IgA, IgM and light chains on a nitrocellulose-based dot-blot system, and found to be an IgGI.

The cell line 17C6 is stable in continuous culture. Since the second subclone, there has been no evidence of overgrowth by non-secreting cells or diminution of antibody production. The line has been re-subcloned twice and all resulting subclones on both occasions secreted identical amounts of antibody. The cell line has been successfully frozen, thawed back and continued producing normal amounts of antibody. Attempts to destabilise the line through "stress" have failed, with the line secreting similar levels of antibody in 2.5-20% FCS. Using the batch of FCS in which the fusion was performed, no variation in levels of antibody secretion was detected. However, over a comparable time period in Ultroser serum-free medium, secretion rates decreased, but were regained on returning to serum-containing media. The effect is thus on the cells' environment rather than a change within the cell. Other hybridoma lines, murine and human, have been shown to secrete comparable amounts of immunoglobulin in both serum and serum-free media. This suggests that chemically-defined media which support both murine and human hybridoma growth and secretion rates comparable with serum can be modified to enable ovine lines to be grown under such conditions. The required additional factor required for this cell line is not found in mouse feeder cells.

Other attempts to destabilise the cell line involved subcloning in the absence of growth factors or feeder cells. The cell line subcloned well under these conditions, withstanding the sort of stress many sturdy murine lines cannot endure. The cell line has shown no requirement to go through an adaption phase to withstand shearing forces when transferred from static to spinner cultures in 2.5% FCS, unlike some murine lines which need acclimatisation.

The following Example 2 illustrates humanisation of the high affinity monoclonal antibody of Example 1.

### Example 2

The antibody of Example 1 is subjected to humanisation by any of the known techniques described above. The product is an antibody embodying the present invention, suitable for administration to humans.

**TABLE 1**

| CULTURE CONDITIONS | NUMBER OF HYBRIDOMAS | |
|---|---|---|
| | NS2 fusion partner | SEP1 fusion partner |
| i | 43 | 128 |
| ii | 12 | 145 |
| iii | 123 | 165 |
| iv | 48 | 196 |

**TABLE 2**

| | FCS | | | | MIX | | | | LAMB SERUM | | | |
|---|---|---|---|---|---|---|---|---|---|---|---|---|
| | 5 | 10 | 15 | 20 | 5 | 10 | 15 | 20 | 5 | 10 | 15 | 20 |
| 48h | 9.9% | 9.8% | 9.4% | 9.7% | 10.5% | 11.0% | 11.11 | 11.1% | 10.6% | 11.0% | 11.0% | 10.4% |
| 96h | 36.2% | 36.0% | 35.7% | 38.1% | 30.7% | 32.5% | 28.3% | 31.0% | 25.4% | 23.4% | 22.6% | 19.2% |
| 144h. | 83.7% | 87.6% | 81.5% | 85.9% | 59.4% | 61.2% | 51.0% | 46.9% | 43.9% | 44.2% | 47.6% | 39.0% |

**Table 3**

| Showing % Cross Reactivities of Ovine Monoclonal Antibody compared with Polyclonal Antiserum. | | | |
|---|---|---|---|
| | Monoclonal 17C6 | Polyclonal | |
| | | Early (5.10.76) | Late (15.3.89) |
| L-Triiodothyronine (T₃) | 100. | 100. | 100. |
| 3.5¹5¹L-Triiodothyronine (rT₃) | 0.3 | 2.6 | 0.9 |
| D L Thyroxine | 0.4 | 0.65 | 0.6 |
| 3,5 Diiodo-L-Tyrosine | >0.001 | 0.16 | 0.09 |
| L-Thyroxine | 0.155 | 0.15 | 0.14 |
| D-Thyroxine | 0.07 | 0.25 | 0.2 |

## Claims

1. A chimeric monoclonal antibody comprising the constant region from human immunoglobulin and the variable region from an ovine monoclonal antibody or monoclonal antibody from a cow having an affinity of at least 10^11 l/mol.

2. An antibody according to claim 1, wherein said affinity is at least 10^12 l/mol.

3. An antibody according to claim 1, wherein said affinity is at least 5 x 10^12 l/mol.

4. An antibody according to claim 1, wherein said affinity is at least 10^13 l/mol.

5. An antibody fragment which is an F(ab')2 or Fab fragment of an antibody according to claim 1, in which at least part is derived from human immunoglobulin.

6. An antibody fragment according to claim 5 wherein said affinity is at least 10^12 l/mol.

7. An antibody fragment according to claim 5 wherein said affinity is at least 5 x 10^12 l/mol.

8. An antibody fragment according to claim 5 wherein said affinity is at least 10^13 l/mol.

9. The use of an antibody according to any of claims 1 to 4 for the manufacture of a medicament for use in treating a human subject hosting an antigen to which the antibody binds.

10. The use of an antibody fragment according to any of claims 5 to 8 for the manufacture of a medicament for use in treating a human subject hosting an antigen to which the antibody fragment binds.

## Patentansprüche

1. Chimärer monoklonaler Antikörper, welcher die konstante Region aus menschlichen Immunoglobulin und die variable Region aus einem Schaf-monoklonalen Antikörper oder eines monoklonalen Antikörpers einer Kuh aufweist, mit einer Affinität von zumindest 10¹¹ l/mol.

2. Antikörper nach Anspruch 1, wobei die besagte Affinität zumindest 10¹² l/mol ist.

3. Antikörper nach Anspruch 1, wobei die besagte Affinität zumindest 5 x 10¹² l/mol ist.

4. Antikörper nach Anspruch 1, wobei die besagte Affinität zumindest 10¹³ l/mol ist.

5. Antikörperfragment, welches ein F(ab')2 oder Fab Fragment eines Antikörpers nach Anspruch 1 ist, in welchem zumindest ein Teil von menschlichem Immunglobulin abgeleitet ist.

6. Antikörperfragment nach Anspruch 5, wobei die besagte Affinität zumindest 10¹² l/mol ist.

7. Antikörperfragment nach Anspruch 5, wobei die besagte Affinität zumindest 5 x 10¹² 1/mol ist.

8. Antikörperfragment nach Anspruch 5, wobei die besagte Affinität zumindest 10¹³ l/mol ist.

9. Verwendung eines Antikörpers nach einem der Ansprüche 1 bis 4 für die Herstellung eines Medikamentes zur Verwendung zum Behandeln eines menschlichen Lebewesens, das ein Antigen aufweist, an welches der Antikörper bindet.

10. Verwendung eines Antikörperfragments nach einem der Ansprüche 5 bis 8 für die Herstellung eines Medikamentes zur Verwendung zum Behandeln eines menschlichen Lebewesens, das ein Antigen aufweist, an welches das Antikörperfragment bindet.

## Revendications

1. Anticorps monoclonal chimérique comprenant la région constante d'une immunoglobuline humaine et la région variable d'un anticorps monoclonal ovin ou d'un anticorps monoclonal d'une vache ayant une affinité d'au moins 10¹¹l/mole.

2. Anticorps selon la revendication 1, dans lequel ladite affinité est d'au moins 10¹² l/mole.

3. Anticorps selon la revendication 1, dans lequel ladite affinité est d'au moins 5 x 10¹² l/mole.

4. Anticorps selon la revendication 1, dans lequel ladite affinité est d'au moins 10¹³ l/mole.

5. Fragment d'anticorps qui est un fragment F(ab')2 ou Fab d'un anticorps selon la revendication 1, dans lequel au moins une partie est dérivée d'une immunoglobuline humaine.

6. Fragment d'anticorps selon la revendication 5, dans lequel ladite affinité est d'au moins 10¹² l/mole.

7. Fragment d'anticorps selon la revendication 5, dans lequel ladite affinité est d'au moins 5 x 10¹² l/mole.

8. Fragment d'anticorps selon la revendication 5, dans lequel ladite affinité est d'au moins 10¹³ l/mole.

9. Utilisation d'un anticorps selon l'une quelconque des revendications 1 à 4 pour la fabrication d'un médicament à utiliser dans le traitement d'un sujet humain portant un antigène auquel se lie l'anticorps.

10. Utilisation d'un fragment d'anticorps selon l'une quelconque des revendications 5 à 8 pour la fabrication d'un médicament à utiliser dans le traitement d'un sujet humain portant un antigène auquel se lie le fragment d'anticorps.
